# EUROPÄISCHE PATENTANMELDUNG

(11) **EP 1 927 853 A2**
(43) Veröffentlichungstag der Anmeldung: **04.06.2008**
(21) Anmeldenummer: 07022911.7
(22) Anmeldetag: 27.11.2007
(51) Int. Cl.: G01N 27/74

(54) **Paramagnetischer Sauerstoffsensor**

(30) Priorität: 28.11.2006 DE 102006056046
(71) Anmelder: ABB PATENT GmbH, 68526 Ladenburg (DE)
(72) Erfinder: Krippner, Peter, Dr.-Ing., 76199 Karlsruhe (DE); Ruzzu, Antonio, Dr., 76227 Karlsruhe (DE); Wetzko, Manfred, Dr., 69198 Schriesheim (DE); Bauer, Thomas, Dipl.-Phys., 61350 Bad Homburg (DE); Andres, Berthold, Dr., 63599 Biebergemünd (DE)
(74) Vertreter: Schmidt, Karl Michael

(57) **Zusammenfassung**

Paramagnetischer Sauerstoffsensor mit einer zwischen einander gegenüberliegenden magnetischen Polschuhen (5a, 5b) angeordneten, das Messgas enthaltenen Messkammer (6), in welcher ein Probenkörper (1) um die Achse mindestens einer Aufhängung (2) verschwenkbar angeordnet ist, wobei mindestens ein piezoresistives Element (7a, 7b) eine durch den Messeffekt hervorgerufene elastische Verformung im Bereich der Aufhängung (2) erfasst, um die hieraus resultierende Materialdehnung in eine Änderung des elektrischen Widerstandswertes des piezoresistiven Elements (7a, 7b) umzuwandeln, woraus signalverarbeitungstechnisch die Sauerstoffkonzentration im Messgas ermittelbar ist. Daneben ist auch eine Messung über eine elektrische Kapazitätsänderung möglich.

## Beschreibung

Die vorliegende Erfindung betrifft einen paramagnetischen Sauerstoffsensor, mit einer zwischen einander gegenüberliegenden magnetischen Polschuhen angeordneten, das Messgas enthaltenen Messkammer, in welcher ein Probenkörper um die Achse mindestens einer Aufhängung verschwenkbar angeordnet ist.

Zur messtechnischen Bestimmung von Sauerstoffkonzentrationen in Gasen finden unterschiedliche Messverfahren Anwendung, die von dem jeweils zu erfassenden Konzentrationsbereich sowie den begleitenden Substanzen abhängen. Unterschieden wird prinzipiell in physikalische und chemische Messverfahren. Zu den hier interessierenden physikalischen Messverfahren zählt u.A. das paramagnetische Verfahren. Es geht von der Tatsache aus, dass die Sauerstoffmoleküle aufgrund ihres permanent magnetischen Di-Polmoments paramagnetisch sind, alle anderen Gase mit geringeren Ausnahmen diamagnetisch sind. Als paramagnetisch werden Substanzen bezeichnet, welche eine positive magnetische Suszeptibilität besitzen. Paramagnetische Materialien magnetisieren sich in einem externen Magnetfeld so, dass sie das Magnetfeld in ihrem Inneren effektiv verstärken. Zur messtechnischen Umsetzung wird die Tatsache ausgenutzt, dass ein Sauerstoffmolekül eine Kraft längs des Feldstärkegradienten eines Magnetfeldes in Richtung zu hohen Feldstärken erfährt. Hierdurch kommt es zu einer Anreicherung des Sauerstoffs, beispielsweise zwischen den Polschuhen eines magnetischen Kreises.

Aus der EP 0 614 083 B1 geht ein paramagnetischer Sensor hervor, welcher direkt die Änderung der Eigenschaften eines magnetischen Kreises durch die Anwesenheit von paramagnetischem Sauerstoff in einem oder mehreren Luftspalten auswertet.

Problematisch ist hier, eine ausreichend geringe Drift zu gewährleisten, da beispielsweise thermische Effekte die Eigenschaften des Magnetkreises im Vergleich zu dem relativ schwachen Messeffekt durch den Sauerstoff stark ändern.

Bei einem in der DE 198 03 990 A1 offenbarten anderen paramagnetischen Sensor wird das sich ändernde Schwingungsverhalten eines Probenkörpers im Luftspalt durch die sich ändernde Gasdichte messtechnisch ausgewertet. Das hier angewandte Messverfahren nutzt das Feld eines Permanent- oder Elektromagneten nur zur Konzentration des Sauerstoffes im Luftspalt. Die Erfassung des Messsignals erfolgt über den sekundären Effekt des Schwingungsverhaltens, welcher durch die Konzentrationserhöhung beeinflusst wird. Die Unterscheidung der durch die Sauerstoffanreicherung hervorgerufenen Änderung gegenüber der von anderen mit schwankender Konzentration vorhandenen Gase gestaltet sich hier als schwierig und führt zu Querempfindlichkeiten.

Aus der DE 23 01 825 A1 geht ein weiteres paramagnetisches Messverfahren hervor, bei welchem ein Probenkörper von der unterschiedlichen Dichte des Gases im Luftspalt, worin eine hohe Sauerstoffkonzentration herrscht, gegenüber der Dichte des umgebenden Gasraums verdrängt wird. Die resultierende Auslenkung des Probenkörpers ist ein Maß für die Sauerstoffkonzentration. Insbesondere werden hier Aufbauten eingesetzt, bei denen ein hantelförmiger Probenkörper derart aufgehängt ist, dass er sich um eine Achse, die in seinem Schwerpunkt liegt, drehen kann. Ein am Probenkörper angebrachter Spiegel ermöglicht über eine sogenannte Lichtwaage eine hochempfindliche Lagedetektion. Eine auf den Probenkörper angebrachte Spule ermöglicht es, im Magnetfeld eine Rückstellkraft auf die Hantel aufzubringen. Ein linearer Zusammenhang zwischen erfasstem Signal und Sauerstoffkonzentration ergibt sich, da als Messgröße der Strom ausgewertet wird, welcher benötigt wird, um den Probenkörper in seine Ausgangslage ohne Anwesenheit von Sauerstoff zurückzubewegen.

Dieses Messverfahren ermöglicht zwar eine hochempfindliche Messung des Sauerstoffgehalts; nachteilig ist jedoch, dass der Sauerstoffsensor aufgrund des mit konventionellen Methoden hergestellten Probenkörpers keine kurze Ansprechzeiten erreicht. Dies wird einerseits durch die große Trägheit des Probenkörpers verursacht, andererseits ist aufgrund der Größe des Probenkörpers auch das umgebende Gasvolumen entsprechend groß, so dass der Gasaustausch der Messkammer mit dem zu messenden Gasvolumen relativ langsam erfolgt. Dies wird vermieden, wenn die Messkammer vom Messgas mit ausreichend großer Geschwindigkeit durchströmt wird, dies erzeugt dabei wiederum Messfehler, da die Strömung ebenfalls die Auslenkung des hantelförmigen Probenkörpers beeinflusst. Die Herstellung des Probenkörpers, seine Aufhängung und Justage erfordern ferner eine Vielzahl manueller Fertigungsschritte, die eine effiziente Produktion vereiteln.

Die DE 100 53 314 A1 offenbart einen gattungsgemäßen paramagnetischen Sauerstoffsensor, der die vorstehend erläuterten Nachteile teilweise dadurch umgeht, in dem ein durch mikrotechnische Ätzverfahren hergestellter, monolithischer Sensorchip alle mechanischen Funktionen in sich vereinigt. Diese Lösung erlaubt eine effiziente Massenfertigung dessen Sauerstoffsensors, der bislang nur durch komplizierte feinmechanische Handarbeit zu fertigen war. Nachteilig ist bei dieser technischen Lösung jedoch, dass die Auswertung des durch den Sauerstoff ausgeübten Drehmoments - wie bei herkömmlichen Sauerstoffsensoren auch - über eine Lichtwagenanordnung erfolgt. Hierzu sind zusätzliche, meist optische Bauelemente notwendig, die zudem relativ zum eigentlichen Sensorchip exakt justiert werden müssen.

Es ist daher die Aufgabe der vorliegenden Erfindung, einen paramagnetischen Sauerstoffsensor zu schaffen, welcher mit wenigen einzelnen Bauelementen auskommt, und sich auch als monolithischer Sensorchip fertigungstechnisch umsetzen lässt.

Die Aufgabe wird ausgehend von einem paramagnetischen Sauerstoffsensor gemäß den Merkmalen der unabhängigen Ansprüche 1 oder 11, in Verbindung mit dessen jeweiligen kennzeichnenden Merkmalen gelöst. Die jeweils rückbezogenen abhängigen Ansprüche geben vorteilhafte Weiterbildungen der Erfindung wieder.

Die in beiden nebengeordneten Ansprüchen dargestellte technische Lösung basiert auf dem gemeinsamen Gedanken, dass der Grad der in Folge des Messeffekts hervorgerufenen Abweichung der Lage des Probenkörpers von seiner Solllage ausgewertet wird, und zwar über die physikalischen Sekundäreffekte einer mechanischen Spannungsänderung bzw. eine elektrischen Kapazitätsänderung.

Ersterenfalls erfasst mindestens ein piezoresistives Element eine durch den Messeffekt hervorgerufene elastische Verformung im Bereich der Aufhängung des Probenkörpers, um die hieraus resultierende Materialdehnung in eine Änderung des elektrischen Widerstandswerts des piezoresistiven Elements umzuwandeln, woraus signalverarbeitungstechnisch die Sauerstoffkonzentration im Messgas ermittelbar ist. Im Prinzip kann bei der Realisierung als Sensorchip der im dotierten Silizium auftretende piezoelektrische Effekt ausgenutzt werden. Dieser beruht auf einem sich ändernden Widerstand des dotierten Siliziums bei sich ändernden Materialspannungen. Durch beispielsweise Implantation oder Diffusion eingebrachte Messwiderstände kann so lokal begrenzt über die Auslesung des Widerstands die Spannung im Material ermittelt werden. Diese ist ein Maß für den am membranartigen Probenkörper anliegenden Druck, und letztlich ein Maß für die Sauerstoffkonzentration im Messgas.

Im Falle der kapazitiven Messwerteaufnahme bildet der verschwenkbare Probenkörper mit einem demgegenüber ortsfesten Halterahmen einen elektrischen Kondensator, wobei die aus der Verschwenkung resultierende Änderung der elektrischen Kapazität erfasst wird, woraus signalverarbeitungstechnisch ebenfalls die Sauerstoffkonzentration in Messgas ermittelbar ist. Bei dieser Lösung wird im Falle einer Realisierung als Sensorchip der Umstand ausgenutzt, dass die Deformation des Siliziums zu einer Änderung eines Kondensatorspalts führt. Durch entsprechende Kontaktflächen auf den Siliziumstrukturen, die die Elektroden dieses Kondensators bilden, wird die sich ändernde Kapazität elektrisch ausgelesen. Diese ist dabei ein Maß für den an dem membranförmigen Probenkörper anliegenden Druck, letztlich also ein Maß für die Sauerstoffkonzentration im Messgas.

Die gerätetechnische Umsetzung der beiden erfindungsgemäßen Lösungsalternativen vereinfacht den Aufbau von paramagnetischen Sauerstoffsensoren erheblich. Dabei bleibt der klassische Messeffekt, bei dem auf einen Probenkörper im inhomogenen Magnetfeld durch sich ändernden Sauerstoffgehalt in der umgebenden Atmosphäre ein Drehmoment wirkt, erhalten. Wesentlicher Unterschied zum herkömmlichen Messverfahren, bei dem die resultierende Drehbewegung durch eine Lagedetektion mittels Lichtwaage ausgelesen wird, ist nun die direkte elektrische Auslesung der Materialspannung in der Aufhängung des Probenkörpers bzw. der elektrischen Kapazität sich ändernder Kondensatorplatten.

Im Falle der Verwendung eines piezoresitiven Elements zur Messwerterfassung sollte dieses vorzugsweise in dem Bereich der Aufhängung angeordnet sein, in welchem eine maximale Materialdehnung stattfindet. Denn an dieser Stelle ergibt sich der größte Messeffekt. In diesen Bereichen maximaler Materialdehnung werden durch in das Sensormaterial eingebrachte piezoresistive Elemente die Dehnungswerte in Änderungen des elektrischen Widerstands umgesetzt.

Zur Erzielung eines besonders hohen Messeffekts wird vorgeschlagen, die Aufhängung zur Herbeiführung einer entsprechend hohen Materialdehnung mit einer örtlichen Spannungskonzentrationseinheit auszustatten. Diese führt die Konzentration der Verformung an gewünschten Stellen der Aufhängung herbei. An diesen Stellen erzeugter maximaler Materialdehnung werden dann die piezoresistiven Elemente als Sensoren eingebracht.

Vorzugsweise wird die Spannungskonzentrationseinheit aus einer Verjüngungsstelle der Aufhängung gebildet, worin vorzugsweise genau zwei piezoresistive Elemente radial beabstandet zueinander angeordnet sind, in welchen bei Verschwenken der Aufhängung gegensinnige Spannungen auftreten. Diese lassen sich signalverarbeitungstechnisch eindeutig auswerten. Die radiale Beanstandung zwischen den beiden piezoresistiven Elementen wird vorzugsweise über einen dazwischenliegenden Spalt herbeigeführt, der eine Art Graben zwischen den beiden Piezowiderständen bildet.

Zur Optimierung des als elektrisches Signal vorliegenden Messeffekts können die piezoresistiven Elemente mit einer Brückenschaltung versehen werden.

Gemäß einer bevorzugten Ausführungsform der Erfindung ist der Probenkörper aus mehreren symmetrisch zueinander angeordneten Teilkörpern gleicher Gestalt gebildet, wobei der Schwerpunkt des Probenkörpers im durch die Aufhängung definierten Schwerpunkt des Systems liegt. Ganz vorzugsweise kann ein derartiger Probenkörper kreuzförmig gestaltet werden und in seinem Schwerpunkt durch eine zweiachsige Aufhängung gehalten sein. Dabei ist die zweiachsige Aufhängung vorzugsweise aus zwei zueinander orthogonal verlaufenden Aufhängungsachse gebildet, die sich von einem äußeren Halterahmen aus nach innen erstreckt. Diese bevorzugte Ausführungsform eignet sich besonders, um den erfindungsgemäßen paramagnetischen Sauerstoffsensor mit piezoelektrischer Auswertung in Form eines Silizium-Chips herzustellen.

Bei der Umsetzung eines Silizium-Sensor beläuft sich die Höhe der Struktur im Bereich der Spannungskondensatoreinheit vorzugsweise im Bereich von 10-500 Mikrometern. Die Aufhängungsstege weisen eine typische Breite von 100 Mikrometern auf, wobei die Verjüngungen an der Stelle der Spannungskonzentrator-Einheit im Bereich von 10-20 Mikrometer liegen sollten. Somit lassen sich Außenabmessungen für den Siliziumsensor realisieren, welche im Bereich von 10 x 10 mm² - 20 x 20 mm² liegen.

Ist der paramagnetische Sauerstoffsensor nach der Variante der kapazitiven Auswertung ausgebildet, so lässt sich eine ähnliche Struktur umsetzen. Eine zweiachsige Aufhängung aus zwei zueinander orthogonal verlaufenden Aufhängungsachsen können ebenfalls von einem äußeren Halterahmen aus nach innen geführt werden. Der Probenkörper selbst kann vorzugsweise ebenfalls kreuzförmig ausgebildet sein, wobei der Halterahmen zur Bildung eines elektrischen Kondensators gemeinsam mit dem verschwenkbaren Probekörper Anformungen zur Ausbildung entsprechender Kondensatorspalte erhält. Eine vorteilhafte Breite dieser Kondensatorspalte liegt im Bereich von 10-50 Mikrometern. Die Breite der Aufhängung dieser kapazitiven Variante weist eine typische Breite von vorzugsweise 10-20 Mikrometern auf.

Damit Verunreinigungen im Messgas sich nicht in den engen Kondensatorspalten absetzen können, wird gemäß einer weiteren, die Erfindung verbessernden Maßnahme vorgeschlagen, die Anordnung der Kondensatorspalte von den eigentlichen Probenkörpern in der Messkammer abzutrennen. Dies kann beispielsweise durch Dichtlippen erfolgen, die eine Bewegung der Anordnung aus Probenkörper und Kondensatorplatten zulassen, das Eindringen von Partikeln in den Bereich der Kondensatoren jedoch verhindern. Kurzschlüsse oder eine mechanische Blockade können somit vermieden werden.

Weitere, die Erfindung verbessernde Maßnahmen werden nachstehend gemeinsam mit der Beschreibung bevorzugter Ausführungsformen der Erfindung in beiden Varianten näher dargestellt. Es zeigt:
- Fig.1: eine Draufsicht auf eine Anordnung mit Probenkörper, Aufhängung und Halterahmens eines paramagnetischen Sauerstoffsensors in einer ersten Variante,
- Fig.2: einen Längsschnitt der Anordnung gemäß Fig.1 entlang der Schnittlinie A-A,
- Fig.3: ein Detail im Bereich der Aufhängung des Probenkörpers gemäß Fig.1 in einer ersten Ausführungsform,
- Fig.4: ein Detail der Aufhängung des Probenkörpers gemäß Fig.1 in einer weiteren Ausführungsform, und
- Fig.5: eine Draufsicht einer Anordnung mit Probenkörper, Aufhängung und Halterahmen in einer weiteren Variante der Erfindung.

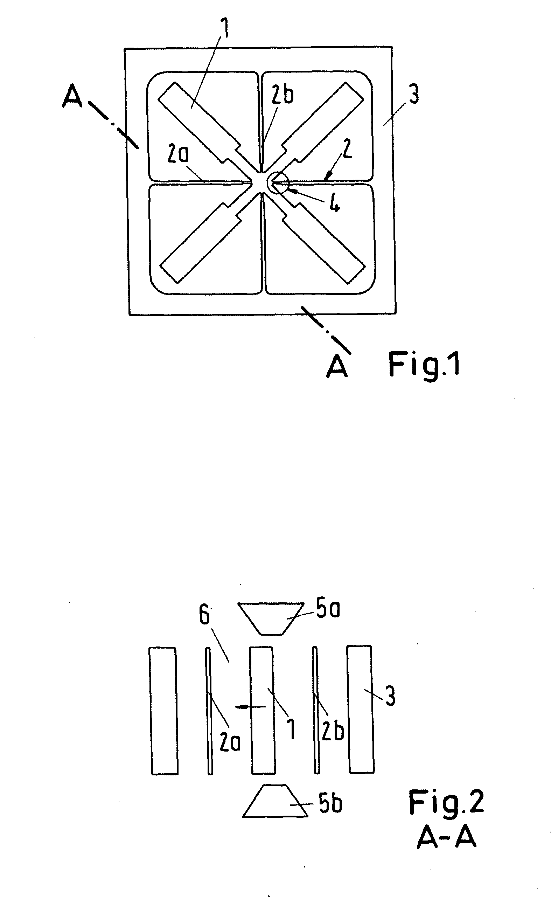

Die in Fig.1 dargestellte Anordnung ist Bestandteil eines paramagnetischen Sauerstoffsensors, dessen Messwertermittlung über die Erfassung und Auswertung von durch den Messeffekt hervorgerufenen elastischen Verformungen erfolgt. Ein hier kreuzförmig gestalteter Probenkörper 1 ist in seinem Schwerpunkt durch eine zweiachsige Aufhängung 2 gehalten. Der Probenkörper 1 besteht aus zwei symmetrisch zueinander angeordneten Teilkörpern gleicher Gestalt, wobei der Schwerpunkt des Probenkörpers 1 im durch die Aufhängung definierten Schwerpunkt des Systems liegt. Die zweiachsige Aufhängung 2 ist demgegenüber aus zwei zueinander orthogonal verlaufenden Aufhängungsachsen 2a, 2b gebildet, welche sich von einem äußeren die Anordnung umgebenden Halterahmen 3 nach innen erstrecken. Die aus Probenkörper 1, Aufhängung 2 sowie Halterahmen 3 bestehende Anordnung ist einstückig ausgebildet, und zwar in Form eines geätzten Silizium-Chips.

Die Aufhängung 2 ist zur Herbeiführung einer hohen Materialdehnung mit einer örtlichen Spannungskonzentratoreinheit 4 ausgestattet, innerhalb welcher ein - hier nicht weiter dargestelltes - piezoresistives Element angeordnet ist.

Gemäß des in Fig. 2 dargestellten Schnitts A-A lässt sich die Funktionsweise des paramagnetischen Sauerstoffsensors erläutern:

Der Probenkörper 1 ist zwischen zwei einander gegenüberliegenden magnetischen Polschuhen 5a und 5b angeordnet. In diesem Bereich befindet sich eine das Messgas enthaltene Messkammer 6. Der Messeffekt äußert sich in einer durch Verschwenken des Probenkörpers 1 hervorgerufenen Verformung im Bereich der Aufhängung 2.

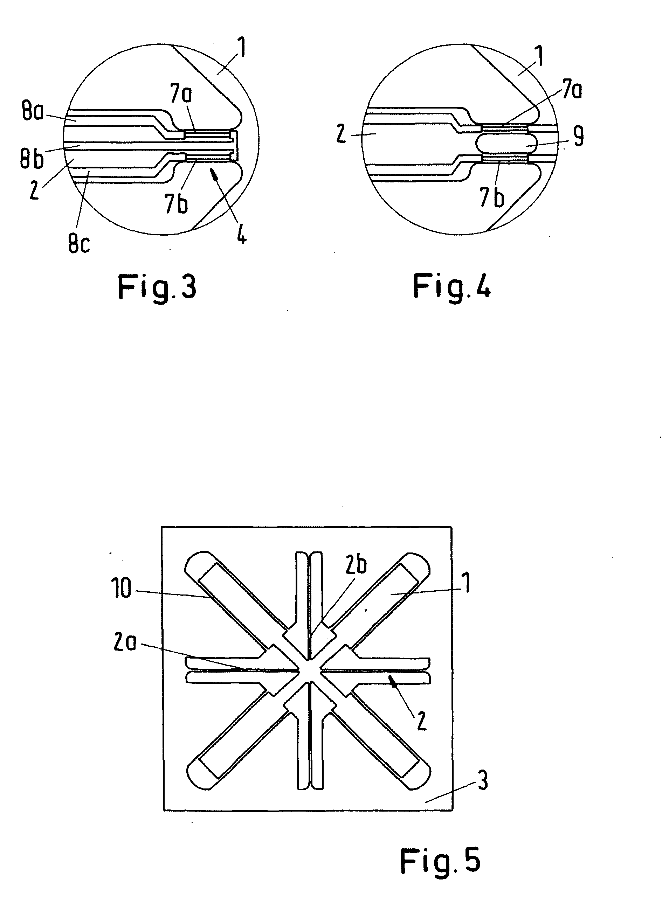

Wie detaillierter aus Fig.3 hervorgeht, ist im Bereich der Spannungskonzentratoreinheit 4 der Aufhängung 2 zwei piezoresistive Elemente 7a und 7b radial beabstandet voneinander angeordnet. Beide piezoresistiven Elemente 7a und 7b stehen mit elektrischen Leiterbahnen 8a bzw. 8b sowie 8c zur Bildung einer - nicht weiter dargestellten - Widerstandsbrücke in Verbindung. Die im Bereich der Spannungskonzentratoreinheit 4 durch den Messeffekt hervorgerufene elektrische Verformung führt eine entsprechende Materialdehnung im Bereich der piezoresitiven Elemente 7a und 7b herbei, welche diese Materialdehnung in einen entsprechenden Widerstandswert umwandeln. Hieraus lässt sich durch weiterführende - nicht weiter dargestellte - signalverarbeitungstechnische Auswertung die Sauerstoffkonuzentration im Messgas ermitteln. Die Spannungskonzentratoreinheit 4 der Aufhängung 2 befindet sich hier im Schnittbereich zum kreuzförmigen Probenkörper 1. Die Spannungskonzentratoreinheit 4 ist aus einer Verjüngung der Aufhängung 2 gebildet, worin die beiden piezoresistiven Elemente 7a und 7b radial beabstandet zueinander angeordnet sind. Durch diese spezielle Anordnung entstehen gegensinnige Spannungen, in den piezoresistiven Elementen 7a und 7b. Die in den piezoresistiven Elementen 7a bzw. 7b erzeugten Zug- bzw. Druckspannungen führen zu einer analogen Vergrößerung bzw. Verkleinerung des Widerstandswerts.

Gemäß der Ausführungsform der Fig.4 wird die radiale Beabstandung zwischen den beiden piezoresistiven Elementen 7a und 7b über einen Spalt 9 realisiert, der eine Art Graben zwischen beiden Elementen bildet. Diese Anordnung führt dazu, dass die Verteilung der Materialspannung gleichmäßiger wird durch unvermeidliche Fertigungstoleranzen bei der Einbringung der piezoresistiven Elemente 7a und 7b mit geringeren resultierenden Toleranzen des elektrischen Ausgangssignals des Sauerstoffsensors zu rechnen ist.

Die in Fig.5 dargestellte Variante der erfindungsgemäßen Lösung setzt eine kapazitive Auswertung des durch das paramagnetische Gas verursachten Drehmoments des Probenkörpers 1 um. Der kreuzförmige Probenkörper 1 ist - genau so wie bei der vorstehend beschriebenen Variante - durch eine zweiachsige Aufhängung 2 gehalten, welche aus zwei zueinander orthogonal verlaufenden Aufhängungsachse 2a und 2b besteht, die von einem äußeren Halterahmen 3 aus sich nach innen erstrecken.

Als Besonderheit dieser Variante ist der Halterahmen 3 jedoch mit sich ebenfalls nach innen erstreckenden Anformungen versehen, welche gemeinsam mit Abschnitten des Probenkörpers 1 einen elektrischen Kondensator bilden. Zu diesem Zwecke besteht zwischen den distalen Enden des Probenkörpers 1 und den modifizierten Anformungsabschnitten des ortsfesten Halterahmens 3 ein Kondensatorspalt 10. Hiermit lässt sich die aus der Verschwenkung des Probenkörpers 1 resultierende Änderung der elektrischen Kapazität erfassen, woraus signalverarbeitungstechnisch die Sauerstoffkonzentration im Messgas ermittelbar ist.

Die Erfindung beschränkt sich nicht auf beiden vorstehend genannten bevorzugten Ausführungsformen. Es sind vielmehr auch Alternativen hiervon denkbar, welche vom Schutzbereich der nachfolgenden Ansprüche umfasst sich. So ist es beispielsweise auch möglich, den paramagnetischen Sauerstoffsensor in beiden Varianten auch in Form eines größeren Messgeräts auszubilden. Die Umsetzung der erfindungsgemäßen Lösung allein in Form von Silizium-Sensoren ist also nicht zwingend.

### Bezugszeichenliste

- 1: Probenkörper
- 2: Aufhängung
- 3: Halterahmen
- 4: Spannungskonzentratoreinheit
- 5: magnetischer Polschuh
- 6: Messkammer
- 7: piezoresistives Element
- 8: Leiterbahn
- 9: Spalt
- 10: Kondensatorspalt

## Patentansprüche

1. Paramagnetischer Sauerstoffsensor mit einer zwischen einander gegenüberliegenden magnetischen Polschuhen (5a, 5b) angeordneten, das Messgas enthaltenen Messkammer (6), in welcher ein Probenkörper (1) um die Achse mindestens einer Aufhängung (2) verschwenkbar angeordnet ist,
**dadurch gekennzeichnet, dass** mindestens ein piezoresistives Element (7a, 7b) eine durch den Messeffekt hervorgerufene elastische Verformung im Bereich der Aufhängung (2) erfasst, um die hieraus resultierende Materialdehnung in eine Änderung des elektrischen Widerstandswertes des piezoresistiven Elements (7a, 7b) umzuwandeln, woraus signalverarbeitungstechnisch die Sauerstoffkonzentration im Messgas ermittelbar ist.

2. Paramagnetischer Sauerstoffsensor nach Anspruch 1,
**dadurch gekennzeichnet, dass** das piezoresistive Element (7a, 7b) in dem Bereich der Aufhängung angeordnet ist, in welchem eine maximale Materialdehnung stattfindet.

3. Paramagnetischer Sauerstoffsensor nach Anspruch 1,
**dadurch gekennzeichnet, dass** die Aufhängung (2) zur Herbeiführung einer hohen Materialdehnung mit einer örtlichen Spannungskonzentratoreinheit (4) ausgestattet ist.

4. Paramagnetischer Sauerstoffsensor nach Anspruch 1,
**dadurch gekennzeichnet, dass** der Probenkörper (1) aus mehreren symmetrisch zueinander angeordneten Teilkörpern gleicher Gestalt besteht, wobei der Schwerpunkt des Probenkörpers (1) im durch die Aufhängung definierten Schwerpunkt des Systems liegt.

5. Paramagnetischer Sauerstoffsensor nach Anspruch 4,
**dadurch gekennzeichnet, dass** der Probenkörper (1) kreuzförmig ausgebildet ist und in seinem Schwerpunkt durch eine zweiachsige Aufhängung (2) gehalten ist.

6. Paramagnetischer Sauerstoffsensor nach Anspruch 5,
**dadurch gekennzeichnet, dass** die zweiachsige Aufhängung (2) aus zwei zueinander orthogonal verlaufenden Aufhängungsachsen (2a, 2b) gebildet ist, die sich von einem äußeren Halterahmen () aus nach Innen erstrecken.

7. Paramagnetischer Sauerstoffsensor nach Anspruch 3,
**dadurch gekennzeichnet, dass** mindestens eine Spannungskonzentratoreinheit (4) pro Aufhängungsachse (2) vorgesehen ist, die sich im Schnittbereich zum kreuzförmigen Probenkörper (1) befindet.

8. Paramagnetischer Sauerstoffsensor nach Anspruch 3,
**dadurch gekennzeichnet, dass** die Spannungskonzentratoreinheit (4) aus einer Verjüngungsstelle der Aufhängung (2) gebildet ist, worin zwei piezoresistive Elemente (7a, 7b) radial beabstandet zueinander angeordnet sind, in welchen bei Verschwenken der Aufhängung (2) gegensinnige Spannungen auftreten.

9. Paramagnetischer Sauerstoffsensor nach Anspruch 8,
**dadurch gekennzeichnet, dass** die radiale Beabstandung zwischen den beiden piezoresistiven Elementen (,) über einen Spalt (9) erfolgt.

10. Paramagnetischer Sauerstoffsensor nach einem der vorstehenden Ansprüche,
**dadurch gekennzeichnet, dass** zur Optimierung des als elektrisches Signal vorliegenden Messeffekts eine Brückenbeschaltung des mindestens einen piezoresistiven Elements (7a, 7b) zum Einsatz kommt.

11. Paramagnetischer Sauerstoffsensor mit einer zwischen einander gegenüberliegenden magnetischen Polschuhen (5a, 5b) angeordneten, das Messgas enthaltenen Messkammer (6), in welcher ein Probenkörper (1) um die Achse mindestens einer Aufhängung (2) verschwenkbar angeordnet ist,
**dadurch gekennzeichnet, dass** der verschwenkbare Probenkörper (1) mit einem demgegenüber ortsfesten Halterahmen (3) einen elektrischen Kondensator bildet, um die aus der Verschwenkung resultierende Änderung der elektrischen Kapazität zu erfassen, woraus signalverarbeitungstechnisch die Sauerstoffkonzentration im Messgas ermittelbar ist.

12. Paramagnetischer Sauerstoffsensor nach Anspruch 11,
**dadurch gekennzeichnet,**
**dass** zur Vermeidung von Störeinflüssen der den elektrischen Kondensator bildende Bereich getrennt ist von der das Messgas enthaltenden Messkammer (6).

13. Paramagnetischer Sauerstoffsensor nach Anspruch 11,
**dadurch gekennzeichnet, dass** der Probenkörper (1) kreuzförmig ausgebildet ist und in seinem Schwerpunkt durch eine zweiachsige Aufhängung (2) gehalten ist.

14. Paramagnetischer Sauerstoffsensor nach Anspruch 13,
**dadurch gekennzeichnet, dass** die zweiachsige Aufhängung (2) aus zwei zueinander orthogonal verlaufenden Aufhängungsachsen (2a, 2b) gebildet ist, die von einem äußeren Halterahmen (3) aus nach Innen erstrecken.
